# EUROPÄISCHE PATENTANMELDUNG

(11) **EP 3 660 001 A1**
(43) Veröffentlichungstag der Anmeldung: **03.06.2020**
(21) Anmeldenummer: 18208511.8
(22) Anmeldetag: 27.11.2018
(51) Int. Cl.: C07D 211/12, C07D 211/70

(54) **VERBESSERTES VERFAHREN ZUR HERSTELLUNG VON TRIACETONDIAMIN UND DESSEN DERIVATEN**

(71) Anmelder: Evonik Operations GmbH, 45128 Essen (DE)
(72) Erfinder: RIEB, Julia, 45772 Marl (DE); MINKE, Katharina, 45130 Essen (DE); NEUMANN, Manfred, 45770 Marl (DE)
(74) Vertreter: Evonik Patent Association

(57) **Zusammenfassung**

Die vorliegende Erfindung betrifft ein verbessertes Verfahren zur Herstellung von Triacetondiamin und dessen alkylsubstituierten Derivaten. Dieses zeichnet sich dadurch aus, dass das Eduktgemisch über einer festen Säure und/oder die Umsetzung in Gegenwart der festen Säure stattfindet.

## Beschreibung

Die vorliegende Erfindung betrifft ein verbessertes Verfahren zur Herstellung von Triacetondiamin (4-Amino-2,2,6,6-tetramethylpiperidin, CAS-Nummer: 36768-62-4; abgekürzt als "TAD") und dessen alkylsubstituierten Derivaten. Dieses zeichnet sich dadurch aus, dass das Eduktgemisch über einer festen Säure vorbehandelt und/ oder die Umsetzung in Gegenwart einer festen Säure erfolgt.

### 1. Hintergrund der Erfindung

TAD und dessen alkylsubstituierte Derivate finden vielseitige Anwendung in verschiedenen Gebieten der Technik. So werden sie als Zwischenprodukte bei der Herstellung von UV-Stabilisatoren für Polymere, zum Beispiel Polypropylen, Polyethylen eingesetzt. Daneben können sie auch als Absorbentien von CO₂ zur Reinigung von Sauergasen eingesetzt werden (EP 1 582 250 A1, EP 2 637 766 A1).

Die Herstellung dieser Verbindungen erfolgt standardmäßig durch reduktive Aminierung von Triacetonamin (2,2,6,6-Tetramethyl-4-piperidinon, CAS-Nummer: 826-36-8; abgekürzt als "TAA") mit Ammoniak bzw. dem entsprechenden Amin (EP 0 302 202 A2; DE 692 20 708 T2; EP 2 085 385 A1; EP 0 857 791 B1). Die CN 108409637 A beschreibt beispielsweise die Synthese von *n*-Butyl-TAD aus TAA und *n*-Butylamin. Daneben können die Alkylderivate des TADs auch durch Reaktion von TAD mit dem entsprechenden Aldehyd oder Keton gewonnen werden (EP 3 048 096 A1).

Den von TAA als Edukt ausgehenden Verfahren des Standes der Technik ist gemein, dass man TAA mit einem Amin (RNH₂; der Rest R ist dabei z. B. ein Kohlenwasserstoffrest oder auch Wasserstoff) umsetzt. Dadurch erhält man intermediär ein Imin, welches in Anwesenheit von molekularem Wasserstoff mittels reduktiver Aminierung an einem Edelmetall- oder Nichtedelmetallkatalysator [M] (zum Beispiel Ru, Pd, Pt, Co, Ni) zum gewünschten Zielprodukt reagiert. Dabei werden hohe (EP 0 857 719 B1) oder niedrige (DE 692 20 708 T2) Wasserstoffpartialdrücke angewandt.

Das Verfahren, in denen TAA mit einem primären Amin umgesetzt wird, was zur Bildung einer Schiffschen Base (= Imin) führt, lässt sich wie im folgenden Reaktionsschema **<1>** gezeigt zusammenfassen (wobei R ein aliphatischer Rest oder Wasserstoff ist und [M] ein Träger- oder Vollkatalysator ist).

Dementsprechend kann TAA auch mit einem sekundären Amin umgesetzt werden. Dies führt zur Bildung eines Enamins und lässt sich wie im folgenden Reaktionsschema **<2>** gezeigt zusammenfassen (wobei R', R" aliphatische Rest sind und [M] ein Träger- oder Vollkatalysator ist).

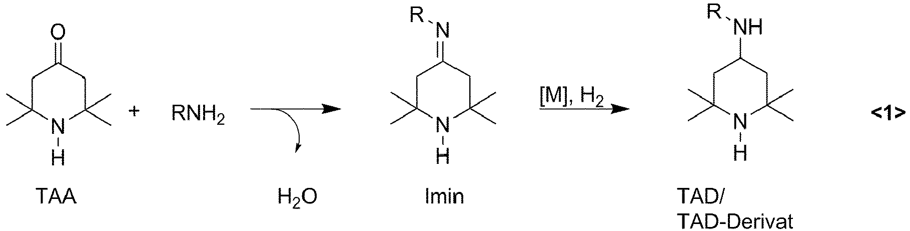

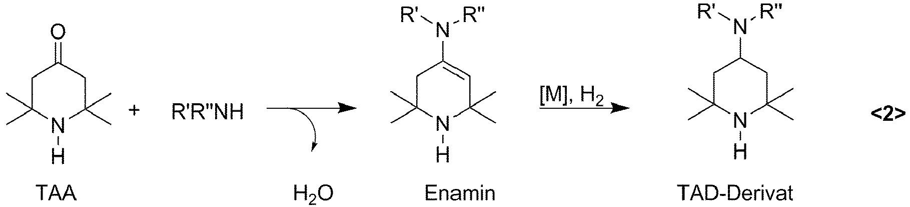

Es besteht weiterhin das Bedürfnis nach verbesserten Verfahren, mit denen die Ausbeute an TAD und dessen alkylsubstituierten Derivaten verbessert werden kann.

Die Aufgabe der vorliegenden Erfindung bestand deshalb darin, ein verbessertes Verfahren zur Synthese von TAD und dessen Derivaten zur Verfügung zu stellen, welches eine gegenüber den herkömmlichen Verfahren effizientere Umsetzung zu TAD und dessen Derivaten erbringt.

### 2. Detaillierte Beschreibung der Erfindung

Es wurde nun überraschend ein Verfahren gefunden, welches die erfindungsgemäße Aufgabe löst.

Es wurde nämlich festgestellt, dass die Gegenwart einer festen Säure während des Verfahrens zu einem effizienteren Umsatz führt.

Das erfindungsgemäße Verfahren ist demnach eines zur Herstellung einer Verbindung der Formel **(I-A)** oder **(I-B)** mit 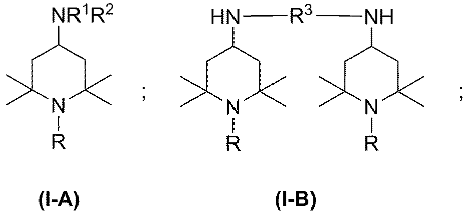 wobei R aus der Gruppe bestehend aus Wasserstoff, Alkylgruppe, Alkoxygruppe ausgewählt ist, und wobei R¹, R² aus der Gruppe bestehend aus Alkylgruppe, Dialkylaminoalkylgruppe ausgewählt sind und R¹ auch Wasserstoff sein kann, und wobei R³ eine Alkylengruppe ist,
umfassend die folgenden Schritte
(a) Umsetzen einer Verbindung der Formel **(II)** 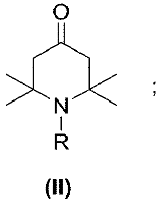 mit mindestens einem Amin, welches aus der Gruppe bestehend aus Verbindungen der der Formel **(III-A)** mit R¹R²NH und Verbindungen der Formel **(III-B)** mit H₂N-R³-NH₂ ausgewählt ist, in einer ersten Stufe zu einer Verbindung der Formel **(IV-A)** bzw. **(IV-B)** bzw. **(IV-C)** mit 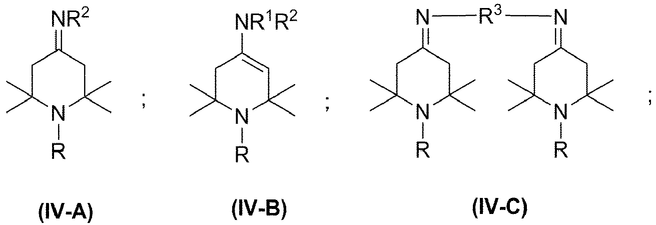
(b) Umsetzen der in Schritt (a) erhaltenen Verbindung der Formel **(IV-A)** bzw. **(IV-B)** bzw. **(IV-C)** mit Wasserstoff in Gegenwart eines Voll- oder Trägerkatalysators **K₁** umfassend mindestens ein elementares Metall ausgewählt aus der Gruppe bestehend aus V, Cr, Mo, Mn, Ni, Pd, Pt, Fe, Ru, Os, Co, Rh, Ir, Cu,
dadurch gekennzeichnet, dass Schritt (a) und/oder Schritt (b) in Gegenwart einer festen Säure **S₁** durchgeführt wird.

Die vorliegende Erfindung beruht auf der überraschenden Erkenntnis, dass die Bildung der Imin- bzw. Enaminintermediate **(IV-A), (IV-B)** bzw. **(I-C)** beschleunigt wird, wenn entsprechend dem Verfahren eine feste Säure **S₁** eingesetzt wird. Die schnellere Bildung der Intermediate führt gleichzeitig zu einer schnelleren Bildung der gewünschten Endprodukte **(I-A)** bzw. **(I-B)** und weniger Rückreaktionen, wodurch die Zersetzung von TAA und Bildung von Zerfallsprodukten des TAA reduziert wird.

Das erfindungsgemäße Verfahren stellt eine reduktive Aminierung dar und wird in zwei Schritten durchgeführt. Im Schritt (a) wird die Carbonylgruppe der Verbindung der Formel **(II)** mit einem Stickstoffatom des Amins **(III-A)** oder **(III-B)** umgesetzt wird. Bei dieser Reaktion wird ein Wasserstoffrest der Amingruppe des Amins **(III-A)** oder **(III-B)** durch den mit dem Sauerstoffatom der Carbonylgruppe verbundenen organischen Rest der Carbonylverbindung **(II)** substituiert.

Wird das Amin **(III-B)** eingesetzt, bildet sich dadurch am Ende des Schrittes (a) als Intermediat die Schiffsche Base der Formel **(IV-C).**

Wird das Amin **(III-A)** eingesetzt und handelt es sich dabei um ein primäres Amin, was der Fall ist, wenn R¹ ein Wasserstoffrest ist, dann bildet sich dadurch am Ende des Schrittes (a) als Intermediat die Schiffsche Base der Formel **(IV-A).**

Wird das Amin **(III-A)** eingesetzt und handelt es sich dabei um ein sekundäres Amin, was der Fall ist, wenn R¹ eine Alkylgruppe oder Dialkylaminoalkylgruppe ist, dann bildet sich dadurch am Ende des Schrittes (a) als Intermediat das Enamin der Formel **(IV-B).**

"[...] Verbindung der Formel **(IV-A)** bzw. **(IV-B)** bzw. **(IV-C)** [...]" in Schritt (a) des erfindungsgemäßen Verfahrens bedeutet demnach, dass, abhängig davon, welche der Amine **(III-A)** oder **(III-B)** und zusätzlich abhängig davon, ob es sich bei dem Amin **(III-A)** um ein sekundäres, was der Fall für R¹ = Alkylgruppe, Dialkylaminoalkylgruppe ist, oder primäres, was der Fall für R¹ = Wasserstoff ist, Amin handelt, als Intermediat eine der Schiffschen Basen **(IV-A)** oder **(IV-C)** oder ein Enamin **(IV-B)** gebildet wird.

Dieses Intermediat wird dann im zweiten Schritt (b) des erfindungsgemäßen Verfahrens mit Wasserstoff in Gegenwart eines Voll- oder Trägerkatalysators **K₁** umfassend mindestens ein elementares Metall ausgewählt aus der Gruppe bestehend aus V, Cr, Mo, Mn, Ni, Pd, Pt, Fe, Ru, Os, Co, Rh, Ir, Cu zum Produkt **(I-A)** bzw. **(I-B)** umgesetzt.

Es hat sich überraschend gezeigt, dass der Anteil des jeweiligen Imin- bzw. Enaminintermediats **(IV-A), (IV-B)** bzw. **(IV-C)** in Schritt (a) schneller erhöht bzw. in Schritt (b) stabilisiert wird, wenn der Schritt (a) bzw. (b) des erfindungsgemäßen Verfahrens in Gegenwart einer festen Säure **S₁** durchgeführt wird.

Dadurch erhöht sich der Anteil der reaktiven Spezies, die in Schritt (b) dann zum gewünschten Produkt **(I-A)** bzw. **(I-B)** weiterreagieren kann, schneller.

Deshalb zeichnet sich das erfindungsgemäße Verfahren dadurch aus, dass Schritt (a) und/oder Schritt (b) in Gegenwart einer festen Säure **S₁** durchgeführt wird.

### 2.1 Schritt (a) des erfindungsgemäßen Verfahrens

Im Schritt (a) des erfindungsgemäßen Verfahrens wird eine Verbindung der Formel **(II)** mit mindestens einem Amin, welches aus der Gruppe bestehend aus Verbindungen der Formel **(III-A)** mit R¹R²NH und Verbindungen der Formel **(III-B)** mit H₂N-R³-NH₂ ausgewählt ist, zu einer Verbindung der Formel **(IV-A)** bzw. **(IV-B)** bzw. **(IV-C)** in Gegenwart einer festen Säure **S₁** umgesetzt.

Die feste Säure **S₁** ist dabei nicht weiter beschränkt, solange sie eine saure LEWIS- oder BRØNSTED-Katalyse der Umsetzung in Schritt (a) gewährleistet. Insbesondere handelt es sich bei der Säure **S₁** um ein Siliciumoxid oder Metalloxid, wobei das Metall bevorzugt aus der Gruppe bestehend aus Aluminium, Titan, Zirkonium ausgewählt ist. Besonders bevorzugt handelt es sich bei der Säure **S₁** um ein Metalloxid ausgewählt aus Al₂O₃, ZrO₂, SiO₂, MgO₂, TiO₂, besonders bevorzugt um Al₂O₃.

Das Verhältnis der molaren Mengen an in Schritt (a) eingesetzten Reaktanden **(II)** und **(III-A)** bzw. **(III-B)** ist grundsätzlich nicht beschränkt.

Es hat sich jedoch als vorteilhaft erwiesen und ist deswegen bevorzugt, dass im Schritt (a) des erfindungsgemäßen Verfahrens für jedes Mol Amin der Formel **(III-A)** 0.25 bis 1.25 Mol, bevorzugt 0.5 bis 1 Mol, bevorzugter 0.83 bis 1 Mol, am bevorzugtesten 0.95 bis 1 Mol der Verbindung **(II)** eingesetzt wird. Die so errechnete molare Menge der Verbindung der Formel **(II)** sei als **T_{(II)}^{A}** abgekürzt.

Zusätzlich dazu wird in dieser bevorzugten Ausführungsform im Schritt (a) des erfindungsgemäßen Verfahrens für jedes Mol Amin der Formel **(III-B)** 0.45 bis 2.5 Mol, bevorzugt 0.5 bis 1.66 Mol, bevorzugter 0.5 bis 0.95 Mol, noch bevorzugter 0.5 Mol bis 0.66 Mol, und am bevorzugtesten 0.5 Mol der Verbindung **(II)** eingesetzt. Die so errechnete molare Menge der Verbindung der Formel **(II)** sei als **T_{(II)}^{B}** abgekürzt.

Es versteht sich von selbst, dass sich die molare Gesamtmenge der in Schritt (a) eingesetzten Verbindung der Formel **(II),** abgekürzt als **T_{(II)},** sich aus der Summe der so in Bezug auf **(III-A)** errechneten Menge an Verbindung der Formel **(II)** und der so in Bezug auf **(III-B)** errechneten Menge an Verbindung der Formel **(II)** ergibt, sich also gemäß **T_{(II)}** = **T_{(II)}^{A}** + **T_{(II)}^{B}** errechnet.

Der Druck in Schritt (a) des erfindungsgemäßen Verfahrens ist nicht beschränkt und liegt insbesondere im Bereich von 1 bar bis 500 bar, bevorzugt im Bereich 10 bar bis 350 bar, bevorzugter im Bereich von 25 bar bis 300 bar.

Die Temperatur in Schritt (a) des erfindungsgemäßen Verfahrens ist nicht beschränkt und liegt bevorzugt im Bereich von 20 °C bis 350 °C, bevorzugter im Bereich von 50 °C bis 150 °C, noch bevorzugter im Bereich von 50 °C bis 120 °C, noch mehr bevorzugter im Bereich von 50 °C bis 80 °C, am bevorzugtesten bei 60 °C bis 70 °C.

Im Schritt (a) des erfindungsgemäßen Verfahrens wird bevorzugt Wasser zugefügt. Es hat sich überraschend gezeigt, dass dies zu einer weiteren Erhöhung der Geschwindigkeit der Bildung der Schiffschen Base bzw. des Enamins führt. Wird Wasser zugegeben, werden bevorzugt zwischen 0.1 bis 1, bevorzugt 0.15 bis 0.8, bevorzugter 0.165 bis 0.7, noch bevorzugter 0.3 bis 0.6 molare Äquivalente an Wasser, bezogen auf die molare Menge an eingesetzter Verbindung der Formel **(II),** zugegeben.

Weiterhin überraschend war, dass diese Verbesserung dann auch erhalten bleibt, wenn zwischen Schritt (a) und Schritt (b) des erfindungsgemäßen Verfahrens das zugegebene Wasser entfernt wird. In einer weiter bevorzugten Ausführungsform des erfindungsgemäßen Verfahrens erfolgt zwischen Schritt (a) und Schritt (b) die Entfernung von Wasser, noch mehr bevorzugter durch Destillation.

### 2.2 Schritt (b) des erfindungsgemäßen Verfahrens

Im Schritt (b) des erfindungsgemäßen Verfahrens wird die in Schritt (a) erhaltene Schiffsche Base **(IV-A)** bzw. **(IV-C)** bzw. das Enamin **(IV-B)** dann mit Wasserstoff in Gegenwart eines Voll- oder Trägerkatalysators **K₁** umfassend mindestens ein elementares Metall ausgewählt aus der Gruppe bestehend aus V, Cr, Mo, Mn, Ni, Pd, Pt, Fe, Ru, Os, Co, Rh, Ir, Cu umgesetzt. Der Katalysator **K₁** ist insbesondere ein Voll- oder Trägerkatalysator umfassend mindestens ein elementares Metall, welches aus der Gruppe bestehend aus Pd, Pt, Ni, Co ausgewählt ist, noch bevorzugter ausgewählt aus Palladium auf Kohleträger, bei dem es sich insbesondere um Aktivkohle handelt, oder Raney-Nickel.

Es versteht sich von selbst, dass der Voll- oder Trägerkatalysator **K₁** von **S₁** verschieden ist.

Als Katalysator können in dem erfindungsgemäßen Verfahren Trägerkatalysatoren mit den elementaren Katalysatormetallen Palladium und/oder Platin und/oder Kobalt und/oder Nickel eingesetzt werden. Als Trägermaterialien für die Trägerkatalysatoren können beispielsweise Aluminiumoxid, das bevorzugt eine spezifischen Oberfläche von 100 bis 350 m²/g aufweist, Silikate, die bevorzugt eine spezifischen Oberfläche von 400 bis 800 m²/g aufweisen, Aluminiumsilikate, die bevorzugt eine spezifische Oberfläche von 200 bis 600 m²/g aufweisen, Kieselgur, der bevorzugt eine spezifische Oberfläche von 2 bis 35 m²/g aufweist, Aktivkohle, die bevorzugt eine spezifischen Oberfläche von 800 bis 1200 m²/g aufweist, Nickel- oder Kobaltoxid, das bevorzugt eine spezifische Oberfläche von 400 bis 900 m²/g aufweist, oder Zeolithe, die bevorzugt eine spezifische Oberfläche von 400 bis 900 m²/g aufweisen, eingesetzt werden. Die in dem erfindungsgemäßen Verfahren bevorzugt eingesetzten Trägerkatalysatoren weisen von 0.5 bis 10 Gew.-%, bevorzugt 0.7 bis 5 Gew.-% mindestens eines elementaren Katalysatormetalls ausgewählt aus der Gruppe bestehend aus Kobalt, Nickel, Platin, Palladium, und besonders bevorzugt Palladium, auf.

Handelt es sich bei dem Katalysator **K₁** um einen Trägerkatalysator, wird dieser bevorzugt in einer Menge von 0.3 bis 15 Gew.-%, bevorzugt von 0.5 bis 12 Gew.-% und besonders bevorzugt von 1 bis 10 Gew.-%, bezogen auf das Gesamtgewicht aller in Schritt (a) eingesetzten Verbindungen der Formel **(II),** eingesetzt.

Handelt es sich bei dem Katalysator **K₁** um einen Vollkatalysator, wird dieser bevorzugt in einer Menge von 1 bis 12 Gew.-% und besonders bevorzugt von 1.5 bis 10 Gew.-%, bezogen auf das Gesamtgewicht aller in Schritt (a) eingesetzten Verbindungen der Formel **(II),** eingesetzt.

Bevorzugt werden als Vollkatalysatoren Katalysatoren vom Typ Raney mit den Aktivmetallen Kobalt und/oder Nickel, noch bevorzugter Nickel, in dem erfindungsgemäßen Verfahren eingesetzt. Insbesondere werden Katalysatoren vom Typ Raney eingesetzt.

Katalysatoren vom Typ Raney sind dem Fachmann bekannt und werden hergestellt, in dem Metalllegierungen eingesetzt werden, die einen Gehalt von 30 bis 60 Gew.-% an Nickel und/oder Kobalt und von 70 bis 40 Gew.-% an Aluminium aufweisen. Durch Herauslösen des Aluminiums wird ein aktiver Katalysator erzeugt, wobei der Rest-Aluminiumgehalt vorzugsweise im Bereich von 2 bis 20 Gew.-% und bevorzugt im Bereich von 5 bis 10 Gew.-% liegt.

Im erfindungsgemäßen Verfahren findet erst eine Umsetzung der Edukte **(II)** und mindestens eine Amins, welches aus der Gruppe bestehend aus **(III-A)** und **(III-B)** ausgewählt ist, zu **(IV-A)** bzw. **(IV-B)** bzw. **(IV-C)** statt. Diese Reaktionsmischung wird dann mit Wasserstoff am Katalysator **K₁** zum jeweiligen Produkt **(II-A)** bzw. **(II-B)** umgesetzt.

In einer bevorzugten Ausführungsform der vorliegenden Erfindung handelt es sich bei dem Amin um eines der Formel **(III-A)** mit R¹R²NH, wobei R¹ und R² jeweils ein Wasserstoffrest sind, und bei R in Formel **(II)** um einen Wasserstoffrest, und bei **K₁** um den Vollkatalysator Raney-Nickel.

In einer bevorzugten Ausführungsform der vorliegenden Erfindung handelt es sich bei dem Amin um eines der Formel **(III-B)** mit H₂N-R³-NH₂, R³ = Hexamethyldiamin und bei R in Formel **(II)** um einen Wasserstoffrest, und bei **K₁** um einen Trägerkatalysator umfassend Pd, wobei der Träger aus der Gruppe bestehend aus Al₂O₃, Kohle, TiO₂ ausgewählt ist.

Die Temperatur in Schritt (b) des erfindungsgemäßen Verfahrens ist nicht weiter beschränkt und liegt bevorzugt im Bereich von 20 °C bis 350 °C, bevorzugter im Bereich von 50 °C bis 300 °C, noch bevorzugter im Bereich von 50 °C bis 250 °C, noch mehr bevorzugter im Bereich von 70 °C bis 220 °C, am bevorzugtesten im Bereich von 80 °C bis 180 °C.

Der Druck in Schritt (b) des erfindungsgemäßen Verfahrens ist nicht weiter beschränkt und liegt bevorzugt im Bereich von 2 bar bis 500 bar, bevorzugter im Bereich von 5 bar bis 350 bar, noch bevorzugter im Bereich von 10 bar bis 350 bar, noch mehr bevorzugter im Bereich von 25 bar bis 300 bar.

Der Schritt (b) kann auch wie im Verfahren gemäß EP 2 085 385 A1 beschrieben durchgeführt werden.

### 2.3 Kennzeichnender Schritt des erfindungsgemäßen Verfahrens

Das erfindungsgemäße Verfahren ist dadurch gekennzeichnet, dass Schritt (a) und/oder Schritt (b) in Gegenwart der festen Säure **S₁** durchgeführt wird. Die feste Säure **S₁** kann beispielsweise bei der Umsetzung der Edukte in Schritt (a) zugegeben werden und/oder in Schritt (b) bei der Umsetzung der Schiffschen Base bzw. des Enamins zum Produkt **(I-A)** bzw. **(I-B).**

Die Gesamtmenge der im erfindungsgemäßen Verfahren eingesetzten Säure **S₁** ist nicht weiter beschränkt und beträgt bevorzugt das 0.5 bis 10, bevorzugter 1 bis 9, noch bevorzugter 2 bis 8, noch mehr bevorzugter 3 bis 7, noch viel mehr bevorzugter 4 bis 6, am bevorzugtesten 5-fachen Menge am im Verfahren eingesetzten Katalysators **K₁.**

Alternativ kann die Gesamtmenge der im erfindungsgemäßen Verfahren eingesetzten Säure **S₁** so eingestellt werden, dass das Verhältnis aus dem Gesamtgewicht der eingesetzten festen Säure **S₁** zum Gesamtgewicht aller eingesetzten Verbindungen der Formel **(II)** im Bereich 0.1 : 1 bis 50 : 1, bevorzugt im Bereich 0.16 : 1 bis 20 : 1, bevorzugter im Bereich 0.5 : 1 bis 10 : 1, noch bevorzugter 2 : 1 bis 5 : 1 liegt.

Demnach ergeben sich die folgende bevorzugten Ausführungsformen des erfindungsgemäßen Verfahrens:
In der ersten liegen die feste Säure **S₁** und der Voll- oder Trägerkatalysator **K₁** gemischt vor, und die Edukte **(II)** und **(III-A)** bzw. **(III-B)** werden an dieser Mischung umgesetzt. Dadurch finden Schritt (a) und Schritt (b) des erfindungsgemäßen Verfahrens unmittelbar hintereinander statt und werden beide in Gegenwart der festen Säure **S₁** durchgeführt.

In einer alternativen Ausführungsform wird Schritt (a) des erfindungsgemäßen Verfahrens in Gegenwart der festen Säure **S₁** durchgeführt. Dies ist beispielsweise bei kontinuierlicher Verfahrensweise möglich, bei der ein Eduktstrom die Füllung eines Reaktors aus fester Säure **S₁** durchläuft, wobei bei Durchlaufen der Schritt (a) des erfindungsgemäßen Verfahrens durchgeführt wird, und der so erhaltene Strom umfassend die Schiffsche Base bzw. das Enamin einen mit Katalysator **K₁** gefüllten Bereich durchläuft. In dieser Ausführungsform finden die Schritte (a) und (b) des erfindungsgemäßen Verfahrens räumlich getrennt voneinander statt.

In einer weiteren alternativen Ausführungsform wird Schritt (b) des erfindungsgemäßen Verfahrens in Gegenwart der festen Säure **S₁** durchgeführt. Dies ist beispielsweise bei kontinuierlicher Verfahrensweise möglich, bei der ein Eduktstrom einen Teil eines Reaktors, der keine feste Säure **S₁** enthält, durchläuft, wobei bei Durchlaufen des nicht mit fester Säure **S₁** gefüllten Teils der Schritt (a) des erfindungsgemäßen Verfahrens durchgeführt wird. Der so erhaltene Strom umfassend die Schiffsche Base bzw. das Enamin wird dann einem mit einer Mischung aus Katalysator **K₁** und fester Säure **S₁** gefüllten Bereich zugeführt, wo dann die weitere Reaktion stattfindet.

### 2.4 Allgemeine Vorschriften

### 2.4.1 Edukte

Die in dem erfindungsgemäßen Verfahren eingesetzten Edukte sind die Verbindung der Formel **(II)** 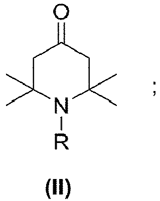 und mindestens ein Amin, welches aus der Gruppe bestehend aus Verbindungen der Formel **(III-A)** mit R¹R²-NH und Verbindungen der Formel **(III-B)** mit H₂N-R³-NH₂ ausgewählt ist.

Dabei ist R aus der Gruppe bestehend aus Wasserstoff, Alkylgruppe, Alkoxygruppe ausgewählt. Bevorzugt ist R aus der Gruppe bestehend aus Wasserstoff, Alkylgruppe mit 1 bis 10, bevorzugter 1 bis 6 Kohlenstoffatomen, Alkoxygruppe mit 1 bis 10, bevorzugter 1 bis 6 Kohlenstoffatomen ausgewählt. Am bevorzugtesten ist R Wasserstoff.

### R¹ ist aus der Gruppe bestehend aus

### Wasserstoff;

Alkylgruppe mit bevorzugt 1 bis 10, bevorzugter 1 bis 6, noch bevorzugter 4, Kohlenstoffatomen, wobei es sich bei der Alkylgruppe am bevorzugtesten um Methyl oder *n*-Butyl handelt, Dialkylaminoalkylgruppe mit bevorzugt 3 bis 10, bevorzugter 3 bis 7, noch bevorzugter 5 bis 6 Kohlenstoffatomen, wobei es sich bei der Dialkylaminoalkylgruppe am bevorzugtesten um 3-Dimethylamino-propylgruppe handelt;
ausgewählt.

R² ist aus der Gruppe bestehend aus
Alkylgruppe mit bevorzugt 1 bis 10, bevorzugter 1 bis 6, noch bevorzugter 4, Kohlenstoffatomen, wobei es sich bei der Alkylgruppe am bevorzugtesten um Methyl oder *n*-Butyl handelt,
Dialkylaminoalkylgruppe mit bevorzugt 3 bis 10, bevorzugter 3 bis 7, noch bevorzugter 5 bis 6 Kohlenstoffatomen, wobei es sich bei der Dialkylaminoalkylgruppe am bevorzugtesten um 3-Dimethylamino-propylgruppe handelt;
ausgewählt.

R³ ist eine Alkylengruppe, bevorzugt mit 1 bis 10 Kohlenstoffatomen, noch bevorzugter 1 bis 6 Kohlenstoffatomen, am bevorzugtesten *n*-Hexylen.

Im Sinne der Erfindung ist eine Alkylgruppe verzweigt oder unverzweigt und weist bevorzugt 1 bis 30, bevorzugter 1 bis 12, noch bevorzugter 1 bis 8, noch mehr bevorzugter 1 bis 6, am bevorzugtesten 1 bis 4, Kohlenstoffatome auf.

Im Sinne der Erfindung ist eine "unverzweigte oder verzweigte Alkylgruppe mit 1 bis 30 Kohlenstoffatomen" insbesondere ausgewählt aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl, *n*-Tridecyl, *n*-Tetradecyl, *n*-Pentadecyl, *n*-Hexadecyl, *n*-Heptadecyl, *n*-Octadecyl, *n*-Nonadecyl, *n*-Eicosyl, *n*-Heneicosyl, *n*-Docosyl, *n*-Tricosyl, *n*-Tetracosyl, *n*-Pentacosyl, *n*-Hexacosyl, *n*-Heptacosyl, *n*-Octocosyl, *n*-Nonacosyl, *n*-Tricontyl.

Im Sinne der Erfindung ist eine "unverzweigte oder verzweigte Alkylgruppe mit 1 bis 12 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl, *n*-Undecyl, *n*-Dodecyl.

Im Sinne der Erfindung ist eine "unverzweigte oder verzweigte Alkylgruppe mit 1 bis 10 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, *n*-Heptyl, *n*-Octyl, *n*-Nonyl, *n*-Decyl.

Im Sinne der Erfindung ist eine "unverzweigte oder verzweigte Alkylgruppe mit 1 bis 8 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl.

Im Sinne der Erfindung ist eine "unverzweigte oder verzweigte Alkylgruppe mit 1 bis 6 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, *n*-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, *n*-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl.

Im Sinne der Erfindung ist eine "unverzweigte oder verzweigte Alkylgruppe mit 1 bis 4 Kohlenstoffatomen" insbesondere ausgewählt aus der Gruppe bestehend aus Methyl, Ethyl, *n*-Propyl, *iso*-Propyl, *n*-Butyl, *sec*-Butyl, *iso*-Butyl, *tert*-Butyl, bevorzugt Methyl, Ethyl, *n*-Butyl, bevorzugter *n*-Butyl.

Eine Alkylengruppe ist ein zweiwertiger verzweigter oder unverzweigter aliphatischer Kohlenwasserstoffrest der allgemeinen Formel -CₓH₂ₓ-. Die Kette an Kohlenstoffatomen "-CₓH₂ₓ" kann dabei linear sein, dann handelt es sich um eine unverzweigte Alkylengruppe. Sie kann aber auch Verzweigungen aufweisen, dann handelt es sich um eine verzweigte Alkylengruppe. Dabei ist x eine ganze Zahl.

x ist bei einer unverzweigten oder verzweigten Alkylengruppe mit 1 bis 30 Kohlenstoffatomen ausgewählt aus dem Bereich 1 bis 30.

x ist bei einer unverzweigten oder verzweigten Alkylengruppe mit 1 bis 12 Kohlenstoffatomen ausgewählt aus dem Bereich 1 bis 12.

x ist bei einer unverzweigten oder verzweigten Alkylengruppe mit 1 bis 6 Kohlenstoffatomen ausgewählt aus dem Bereich 1 bis 6.

Im Sinne der Erfindung ist eine "Alkoxygruppe" unverzweigt oder verzweigt. Es handelt sich dabei um einen organischer Rest der chemischen Struktur - 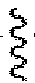 -O-R**,
in welchem R** eine unverzweigte oder verzweigte Alkylgruppe ist. Bei einer "unverzweigten oder verzweigten Alkoxygruppe mit 1 bis 30 Kohlenstoffatomen" ist R** eine unverzweigte oder verzweigte Alkylgruppe mit 1 bis 30 Kohlenstoffatomen.

Bei einer "unverzweigten oder verzweigten Alkoxygruppe mit 1 bis 10 Kohlenstoffatomen" ist R** eine unverzweigte oder verzweigte Alkylgruppe mit 1 bis 10 Kohlenstoffatomen.

Im Sinne der Erfindung ist eine "Alkoxygruppe mit 1 bis 10 Kohlenstoffatomen" insbesondere ausgewählt aus der Gruppe bestehend aus Methoxy, Ethoxy, *n*-Propoxy, *iso*-Propoxy, *n*-Butoxy, *sec-*Butoxy, *iso*-Butoxy, *tert*-Butoxy, *n*-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, *n*-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, *n*-Heptoxy, *n*-Octoxy, *n*-Nonoxy, *n*-Decoxy.

Im Sinne der Erfindung ist eine "Alkoxygruppe mit 1 bis 6 Kohlenstoffatomen" insbesondere ausgewählt aus der Gruppe bestehend aus Methoxy, Ethoxy, *n*-Propoxy, *iso*-Propoxy, *n*-Butoxy, *sec*-Butoxy, *iso*-Butoxy, *tert*-Butoxy, *n*-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, *n*-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy.

Eine Dialkylaminoalkylgruppe ergibt sich aus eine Alkylgruppe durch Substitution eines Wasserstoffatoms der Alkylgruppe durch eine Dialkylaminogruppe, bevorzugt durch eine Dimethylaminogruppe.
Bei einer Dialkylaminoalkylgruppe mit 3 bis 10 bzw. 3 bis 7 bzw. 5 bis 6 Kohlenstoffatomen liegt die Summe aller Kohlenstoffatome (also sowohl den am Aminorest gebundenen als auch den übrigen) bei 3 bis 10 bzw. 3 bis 7 bzw. 5 bis 6.

Die im Sinne der Erfindung bevorzugte Dialkylaminoalkylgruppe ist die 3-Dimethylaminopropylgruppe.

### 2.4.2 Organische Lösungsmittel

Wie vorstehend beschrieben, kann im erfindungsgemäßen Verfahren in Schritt (a) Wasser eingesetzt werden. Es kann auch im Schritt (b) des erfindungsgemäßen Verfahrens Wasser eingesetzt werden.

Das erfindungsgemäße Verfahren kann ohne den Einsatz organischer Lösungsmittel oder auch in mindestens einem organischen Lösungsmittel durchgeführt werden, bevorzugt in mindestens einem organischen Lösungsmittel. Als organische Lösungsmittel eignen sich dabei alle Lösungsmittel, in denen sich die Reaktanden gut lösen und welche keinen störenden Einfluss auf das erfindungsgemäße Verfahren haben. Insbesondere ist das organische Lösungsmittel ausgewählt aus der Gruppe bestehend aus aliphatische Lösungsmittel, aromatische Lösungsmittel, Ether, halogenierte Lösungsmittel, Amide, Thioverbindungen, Carbonsäuren, Alkohole; bevorzugt ist das Lösungsmittel ausgewählt aus der Gruppe bestehend aus aliphatische Lösungsmittel, aromatische Lösungsmittel, Ether, Alkohole; bevorzugter ist das Lösungsmittel ausgewählt aus der Gruppe bestehend aus Ether, Alkohole; noch bevorzugter ist das Lösungsmittel ausgewählt aus der Gruppe bestehend aus Alkohole, noch bevorzugter Methanol oder *n*-Butanol.

Aliphatische Lösungsmittel sind insbesondere ausgewählt aus der Gruppe bestehend aus Pentan, Hexan, Heptan, Octan, Decan, Cyclopentan, Cyclohexan, Methylcyclohexan, Petrolether.

Aromatische Lösungsmittel sind insbesondere ausgewählt aus der Gruppe bestehend aus Benzol, Toluol, Xylol, Ethylbenzol, Cumol, Brombenzol, Chlorbenzol, Dichlorbenzol, Furan.

Ether sind insbesondere ausgewählt aus der Gruppe bestehend aus Diethylether, Dipropylether, Dibutylether, Methyl-*tert*-Butylether, Ethylenglykolmonomethylether, Ethylenglykolmonoethylether, Ethylenglykoldimethylether, Ethylenglykoldiethylether, Diethylenglykolmonomethylether, Diethylenglykolmonoethylether, Diethylenglykoldimethylether, Diethylenglykoldiethylether, Triethylenglykolmonomethylether, Triethylenglykolmonoethylether, Triethylenglykoldimethylether, Triethylenglykoldiethylether, Polyethylenglykolmonomethylether, Polyethylenglykolmonoethylether, Polyethylenglykoldimethylether, Polyethylenglykoldiethylether, 1,4-Dioxan, 1,3-Dioxan, Tetrahydrofuran.

Halogenierte Lösungsmittel sind insbesondere ausgewählt aus der Gruppe bestehend aus Dichlormethan, Chloroform, Tetrachlormethan.

Amide sind insbesondere ausgewählt aus der Gruppe bestehend aus Dimethylformamid, Dimethylacetamid.

Thioverbindungen sind insbesondere ausgewählt aus der Gruppe bestehend aus Dimethylsulfoxid, Sulfolan.

Carbonsäuren sind insbesondere ausgewählt aus der Gruppe bestehend aus Ameisensäure, Essigsäure, Propionsäure, Butansäure, Pentansäure.

Alkohole, sind insbesondere ausgewählt aus der Gruppe bestehend aus
Methanol, Ethanol, Propanol, *iso*-Propanol, 1,2-Propandiol, 1,3-Propandiol, Glycerin, *n*-Butanol, *sec*-Butanol, *iso*-Butanol, *tert*-Butanol, 1,2-Butandiol, 1,3-Butandiol, 1,4-Butandiol, 1-Pentanol, 2-Pentanol, 3-Pentanol, *tert*-Amylalkohol, 1,2-Pentandiol, 1,3-Pentandiol, 1,4-Pentandiol, 1,5-Pentandiol, Cyclopentanol, Hexanol, Cyclohexanol Heptanol, Octanol, Nonanol, Decanol, Ethylenglykol, Diethylenglykol, Triethylenglykol, Polyethylenglykol, Benzylalkohol, Phenol; bevorzugt ausgewählt aus Methanol, Ethanol, *n*-Propanol, *iso*-Propanol, *n*-Butanol, bevorzugter ausgewählt aus Methanol, *n*-Butanol, noch bevorzugter *n*-Butanol.

Wird ein organisches Lösungsmittel eingesetzt, ist es besonders bevorzugt, einen Alkohol einzusetzen.

Ein Arbeiten ohne organisches Lösungsmittel besonders vorteilhaft, wenn eine Verbindung der Formel **(I-B)** hergestellt wird.

### 2.4.3 Reaktionsführung

Das erfindungsgemäße Verfahren kann im Batchbetrieb oder kontinuierlich durchgeführt werden, wird aber bevorzugt im Batchbetrieb durchgeführt.

Bei der Durchführung hat es sich als vorteilhaft erwiesen, die Edukte vor dem Schritt (a) zu mischen und als Mischung in Gegenwart der festen Säure **S₁** zur Verbindung der Formel **(IV-A)** bzw. **(IV-B)** bzw. **(IV-C)** umzusetzen. Diese Ausführungsform des erfindungsgemäßen Verfahrens, bei dem die Edukte, also die Verbindung der Formel **(II)** und das Amin der Formel **(III-A)** mit R¹R²-NH₂ bzw. der Formel **(III-B)** mit H₂N-R³-NH₂ in Schritt (a) als Mischung in Gegenwart einer festen Säure **S₁** zur Verbindung der Formel **(IV-A)** bzw. **(IV-B)** bzw. **(IV-C)** umgesetzt werden, ist deshalb bevorzugt.

### 2.4.4 Bevorzugte Ausführungsformen an Edukten

In einer bevorzugten Ausführungsform handelt es sich bei der Verbindung der Formel **(II)** um Triacetonamin, und bei dem Amin der Formel **(III-A)** um Ammoniak, 3-Dimethylaminopropylamin, *n*-Butylamin, Dimethylamin, und bei dem Amin der Formel **(III-B)** um Hexamethylendiamin.

### 2.4.5 Reinigung der in Schritt (b) erhaltenen Produkte

Nach Durchführung des Schrittes (b) des erfindungsgemäßen Verfahrens erhält man das gewünschte Produkt **(I-A)** bzw. **(I-B).**

Dieses kann mit dem Fachmann geläufigen Methoden weiter gereinigt werden.

Bevorzugt ist eine Reinigungsmethode ausgewählt aus Kristallisation, Destillation.

Es hat sich jedoch die Destillation als besonders vorteilhaft herausgestellt, da mit dieser eine besonders effiziente Reinigung möglich ist. Die konkret anzuwendenden Destillationsbedingungen hängen vom Siedepunkt der jeweiligen Substanz ab und können routinemäßig vom Fachmann bestimmt werden. Bevorzugt wird die Destillation bei einem Druck von 1 bis 50 mbar, bevorzugter 2 bis 40 mbar durchgeführt.

### 3. Beispiele

### 3.1 Umsetzung von Triacetonamin mit n-Butylamin zur entsprechenden Schiffschen Base

### Vergleichsbeispiel V1 mit n-Butylamin (ohne Al₂O₃ und ohne H₂O):

30.1 g Triacetonamin (0.21 mol, CAS-Nummer: 768-66-1) wurden im Kolben vorgelegt und auf 50 °C temperiert. Dann wurden binnen 1 min 15.4 g *n*-Butylamin (0.21 mol, CAS-Nummer: 109-73-9) zugegeben. Die Reaktionsmischung wurde noch weitere 3.5 h bei 50 °C gerührt, jede halbe Stunde eine Probe genommen und nach der Abkühlung der Probe auf Raumtemperatur auf die Zusammensetzung analysiert. Die Analytik erfolgte pur auf einem Gaschromatographen Agilent 6850 auf einer HP50+ Säule. Die Auswertung erfolgte in GC-% area. Tabelle 1 gibt den so ermittelten Anteil an Schiffscher Base des *n*-Butyl-TADs (abgekürzt in der Tabelle 1 als "SB-*n*-Butyl-TAD", entspricht Formel **(IV-A)** mit R = H, R² = *n*-Butyl) wieder.

### Vergleichsbeispiel V2 mit n-Butylamin (ohne Al₂O₃ und mit H₂O):

30.1 g Triacetonamin (0.21 mol, CAS-Nummer: 768-66-1) und 0.6 g H₂O (33.3 mmol) wurden im Kolben vorgelegt und auf 50 °C temperiert. Dann wurden binnen 1 min 15.4 g *n*-Butylamin (0.21 mol, CAS-Nummer: 109-73-9) zugegeben. Die Reaktionsmischung wurde noch weitere 3.5 h bei 50 °C gerührt, jede halbe Stunde eine Probe genommen und nach der Abkühlung der Probe auf Raumtemperatur auf die Zusammensetzung analysiert. Die Analytik erfolgte pur auf einem Gaschromatographen Agilent 6850 auf einer HP50+ Säule. Die Auswertung erfolgte in GC-% area. Tabelle 1 gibt den so ermittelten Anteil an Schiffscher Base des *n*-Butyl-TADs (abgekürzt in der Tabelle 1 als "SB-*n*-Butyl-TAD", entspricht Formel **(IV-A)** mit R = H, R² = *n*-Butyl) wieder.

### Erfinderisches Beispiel E1 mit n-Butylamin (mit Al₂O₃ und H₂O):

30.1 g Triacetonamin (0.21 mol, CAS-Nummer: 768-66-1), 0.6 g H₂O (33.3 mmol) und 5 g Aluminiumoxid (BASF DD-6) wurden im Kolben vorgelegt und auf 50 °C temperiert. Dann wurden binnen 1 min 15.4 g *n*-Butylamin (0.21 mol, CAS-Nummer: 109-73-9) zugegeben. Nach dem Ende der Zugabe wurde die Reaktionsmischung noch weitere 3.5 h bei 50 °C gerührt, jede halbe Stunde eine Probe genommen und nach der Abkühlung der Probe auf Raumtemperatur auf die Zusammensetzung analysiert. Die Analytik erfolgte pur auf einem Gaschromatographen Agilent 6850 auf einer HP50+ Säule. Die Auswertung erfolgte in GC-% area. Tabelle 1 gibt den so ermittelten Anteil an Schiffscher Base des *n*-Butyl-TADs (abgekürzt in der Tabelle 1 als "SB-*n*-Butyl-TAD", entspricht Formel **(IV-A)** mit R = H, R² = *n*-Butyl) wieder.

### Erfinderisches Beispiel E2 mit n-Butylamin (mit Al₂O₃ und ohne H₂O):

30.1 g Triacetonamin (0.21 mol, CAS-Nummer: 768-66-1) und 5 g Aluminiumoxid (BASF DD-6) wurden im Kolben vorgelegt und auf 50 °C temperiert. Dann wurden binnen 1 min 15.4 g *n-*Butylamin (0.21 mol, CAS-Nummer: 109-73-9) zugegeben. Nach dem Ende der Zugabe wird die Reaktionsmischung noch weitere 3.5 h bei 50 °C gerührt, jede halbe Stunde eine Probe genommen und nach der Abkühlung der Probe auf Raumtemperatur auf die Zusammensetzung analysiert. Die Analytik erfolgte pur auf einem Gaschromatographen Agilent 6850 auf einer HP50+ Säule. Die Auswertung erfolgte in GC-% area. Tabelle 1 gibt den so ermittelten Anteil an Schiffscher Base des *n*-Butyl-TADs (abgekürzt in der Tabelle 1 als "SB-*n*-Butyl-TAD", entspricht Formel **(IV-A)** mit R = H, R² = *n*-Butyl) wieder.

**Tabelle 1**

| Zeitpunkt der Probenentnahme | **V1:** Gehalt an SB-*n*-ButylTAD [GC-area%] ohne Al₂O₃, ohne H₂O | **V2:** Gehalt an SB-*n*-ButylTAD [GC-area%] ohne Al₂O₃, mit H₂O | **E2:** Gehalt an SB-*n*-ButylTAD [GC-area%] mit Al₂O₃, ohne H₂O | **E1:** Gehalt an SB-*n*-ButylTAD [GC-area%] mit Al₂O₃, mit H₂O |
|---|---|---|---|---|
| 0 h | 0.00 | 4.82 | 3.00 | 2.78 |
| 0.5 h | 16.43 | 27.94 | 41.21 | 44.94 |
| 1.0 h | 26.68 | 44.59 | 63.43 | 65.99 |
| 1.5 h | 36.33 | 56.73 | 74.89 | 75.33 |
| 2.0 h | 44.02 | 65.73 | 79.90 | 79.34 |
| 2.5 h | n.d | 71.48 | 81.94 | 80.53 |
| 3.0 h | 55.59 | 75.61 | 82.99 | 80.24 |
| 3.5 h | 60.36 | 77.80 | n.d. | n.d |

| | | | | |
|---|---|---|---|---|
| "n.d." = "nicht bestimmt" | | | | |

Die Bildung der Schiffschen Base aus *n*-Butylamin und TAA erfolgt durch die Zugabe von H₂O und Aluminiumoxid **(E2)** wesentlich schneller, wobei die Reaktion mit dem Träger allein, also ohne H₂O **(E1)** am schnellsten verläuft. Da das Gleichgewicht dadurch schneller erreicht wird, kann im Betrieb durch entsprechende Katalysatorschichtung die Raum-Zeit-Ausbeute an Produkt erhöht werden.

### 3.2 Umsetzung von Triacetonamin mit Dimethylamin zum entsprechenden Enamin

Wenn die Beispiele **E1, E2, V1** und **V2** mit Dimethylamin (CAS-Nummer: 124-40-3) an Stelle von *n*-Butylamin wie nachfolgend für die Beispiele **E3, E4, V3** und **V4** beschrieben wiederholt werden, so ist eine Erhöhung der Bildung des entsprechenden Enamins aus Triacetonamin und Diemthylamin [entspricht Formel **(IV-B)** mit R = H, R¹ = Methyl, R² = Methyl] bei den Versuchen **E3** und **E4** gegenüber **V3** bzw. **V4** zu beobachten.

### Vergleichsbeispiel V3:

Vergleichsbeispiel **V1** wird wiederholt, nur dass anstatt 0.21 mol *n*-Butylamin 0.21 mol Dimethylamin eingesetzt werden.

### Vergleichsbeispiel V4:

Vergleichsbeispiel **V2** wird wiederholt, nur dass anstatt 0.21 mol *n*-Butylamin 0.21 mol Dimethylamin eingesetzt werden.

### Erfinderisches Beispiel E3:

Das erfinderische Beispiel **E1** wird wiederholt, nur dass anstatt 0.21 mol *n*-Butylamin 0.21 mol Dimethylamin eingesetzt werden.

### Erfinderisches Beispiel E4:

Das erfinderische Beispiel **E2** wird wiederholt, nur dass anstatt 0.21 mol *n*-Butylamin 0.21 mol Dimethylamin eingesetzt werden.

### 3.3 Umsetzung von Triacetonamin mit Hexamethylendiamin zur entsprechenden Schiffschen Base

Wenn die Beispiele **E1, E2, V1** und **V2** mit Hexamethylendiamin (CAS-Nummer: 124-09-4) an Stelle von *n*-Butylamin wie nachfolgend für die Beispiele **E5, E6, V5** und **V6** beschrieben wiederholt werden, so ist eine Erhöhung der Bildung der entsprechenden Schiffschen Base aus Triacetonamin und Hexamethylendiamin [entspricht Formel **(IV-C)** mit R = H, R³ = Hexylen] bei den Versuchen **E5** und **E6** gegenüber **V5** bzw. **V6** zu beobachten.

### Vergleichsbeispiel V5:

Vergleichsbeispiel **V1** wird wiederholt, nur dass anstatt 0.21 mol *n*-Butylamin 0.105 mol Hexamethylendiamin eingesetzt werden.

### Vergleichsbeispiel V6:

Vergleichsbeispiel **V2** wird wiederholt, nur dass anstatt 0.21 mol *n*-Butylamin 0.105 mol Hexamethylendiamin eingesetzt werden.

### Erfinderisches Beispiel E5:

Das erfinderische Beispiel **E1** wird wiederholt, nur dass anstatt 0.21 mol *n*-Butylamin 0.105 mol Hexamethylendiamin eingesetzt werden.

### Erfinderisches Beispiel E6:

Das erfinderische Beispiel **E2** wird wiederholt, nur dass anstatt 0.21 mol *n*-Butylamin 0.105 mol Hexamethylendiamin eingesetzt werden.

### 3.4 Umsetzung von Triacetonamin mit 3-Aminopropyldimethylamin zur entsprechenden Schiffschen Base

Wenn die Beispiele **E1, E2, V1** und **V2** mit 3-Aminopropyldimethylamin (CAS-Nummer: 109-55-7) an Stelle von *n*-Butylamin wie nachfolgend für die Beispiele **E7, E8, V7** und **V8** beschrieben wiederholt werden, so ist eine Erhöhung der Bildung der entsprechenden Schiffschen Base aus Triacetonamin und 3-Aminopropyldimethylamin [entspricht Formel **(IV-A)** mit R = H, R² = 3-Dimethylaminopropylgruppe] bei den Versuchen **E7** und **E8** gegenüber **V7** bzw. **V8** zu beobachten.

### Vergleichsbeispiel V7:

Vergleichsbeispiel **V1** wird wiederholt, nur dass anstatt 0.21 mol *n*-Butylamin 0.21 mol 3-Aminopropyldimethylamin eingesetzt werden.

### Vergleichsbeispiel V8:

Vergleichsbeispiel **V2** wird wiederholt, nur dass anstatt 0.21 mol *n*-Butylamin 0.21 mol 3-Aminopropyldimethylamin eingesetzt werden.

### Erfinderisches Beispiel E7:

Das erfinderische Beispiel **E1** wird wiederholt, nur dass anstatt 0.21 mol *n*-Butylamin 0.21 mol 3-Aminopropyldimethylamin eingesetzt werden.

### Erfinderisches Beispiel E8:

Das erfinderische Beispiel **E2** wird wiederholt, nur dass anstatt 0.21 mol *n*-Butylamin 0.21 mol 3-Aminopropyldimethylamin eingesetzt werden.

### 3.5 Umsetzung von Triacetonamin mit Ammoniak zur entsprechenden Schiffschen Base

Wenn die Beispiele **E1, E2, V1** und **V2** mit Ammoniak an Stelle von *n*-Butylamin wie nachfolgend für die Beispiele **E9, E10, V9** und **V10** beschrieben wiederholt werden, so ist eine Erhöhung der Bildung der entsprechenden Schiffschen Base aus Triacetonamin und Ammoniak [entspricht Formel **(IV-A)** mit R = H, R² = H] bei den Versuchen **E9** und **E10** gegenüber **V9** bzw. **V10** in situ zu erwarten. Im Falle des Ammoniaks erfolgt der Nachweis der Schiffschen Base indirekt über die Ausbeute an TAD, da die Schiffsche Base schnell zu TAD weiter reagiert.

### Vergleichsbeispiel V9:

Vergleichsbeispiel **V1** wird wiederholt, nur dass anstatt 0.21 mol *n*-Butylamin 0.21 mol gasförmiger Ammoniak eingesetzt werden und bis darauf, dass der Nachweis der Schiffschen Base aus TAA und Ammoniak via GC indirekt über das Endprodukt TAD, zum dem die Schiffsche Base weiterreagiert, erfolgt.

### Vergleichsbeispiel V10:

Vergleichsbeispiel **V2** wird wiederholt, nur dass anstatt 0.21 mol *n*-Butylamin 0.21 mol gasförmiger Ammoniak eingesetzt werden und bis darauf, dass der Nachweis der Schiffschen Base aus TAA und Ammoniak via GC indirekt über das Endprodukt TAD, zum dem die Schiffsche Base weiterreagiert, erfolgt.

### Erfinderisches Beispiel E9:

Das erfinderische Beispiel **E1** wird wiederholt, nur dass anstatt 0.21 mol *n*-Butylamin 0.21 mol gasförmiger Ammoniak eingesetzt werden und bis darauf, dass der Nachweis der Schiffschen Base aus TAA und Ammoniak via GC indirekt über das Endprodukt TAD, zum dem die Schiffsche Base weiterreagiert, erfolgt.

### Erfinderisches Beispiel E10:

Das erfinderische Beispiel **E2** wird wiederholt, nur dass anstatt 0.21 mol *n*-Butylamin 0.21 mol gasförmiger Ammoniak eingesetzt werden und bis darauf, dass der Nachweis der Schiffschen Base aus TAA und Ammoniak via GC indirekt über das Endprodukt TAD, zum dem die Schiffsche Base weiterreagiert, erfolgt.

### 3.6 Umsetzung zum entsprechenden TAD-Derivat

Die in den Versuchen **E1** bis **E10** erhaltenen Reaktionsmischungen werden dann an Trägerkatalysatoren (z.B. Pd auf einem Träger ausgewählt aus Kohle, Al₂O₃, TiO₂) oder Vollkatalysatoren (zum Beispiel Raney-Nickel oder Raney-Cobalt) gemäß den vorbeschriebenen Bedingungen oder dem Fachmann bekannten Bedingungen (z.B. EP 2 085 385 A1) umgesetzt. Dabei wird festgestellt, dass sich die Erhöhung des Anteils der Schiffschen Base oder des Enamins in einer Erhöhung von TAD oder dem entsprechenden TAD-Derivat auswirkt.

## Patentansprüche

1. Verfahren zur Herstellung einer Verbindung der Formel **(I-A)** oder **(I-B)** mit 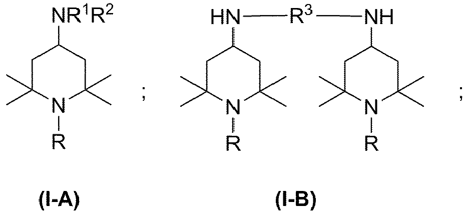 wobei
R aus der Gruppe bestehend aus Wasserstoff, Alkylgruppe, Alkoxygruppe ausgewählt ist,
und wobei R¹, R² aus der Gruppe bestehend aus Alkylgruppe, Dialkylaminoalkylgruppe ausgewählt sind und R¹ auch Wasserstoff sein kann,
und wobei R³ eine Alkylengruppe ist,
umfassend die folgenden Schritte
(a) Umsetzen einer Verbindung der Formel **(II)** 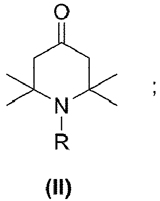 mit mindestens einem Amin, welches aus der Gruppe bestehend aus Verbindungen der Formel **(III-A)** mit R¹R²NH und Verbindungen der Formel **(III-B)** mit H₂N-R³-NH₂ ausgewählt ist, in einer ersten Stufe zu einer Verbindung der Formel **(IV-A)** bzw. **(IV-B)** bzw. **(IV-C)** mit 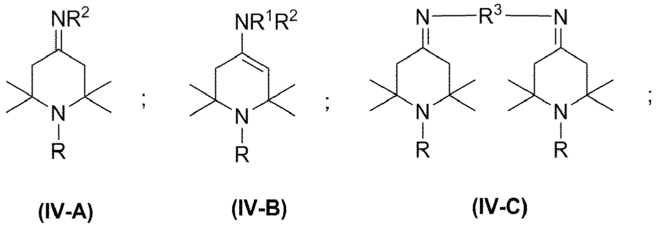
(b) Umsetzen der in Schritt (a) erhaltenen Verbindung der Formel **(IV-A)** bzw. **(IV-B)** bzw. **(IV-C)** mit Wasserstoff in Gegenwart eines Voll- oder Trägerkatalysators **K₁** umfassend mindestens ein elementares Metall ausgewählt aus der Gruppe bestehend aus V, Cr, Mo, Mn, Ni, Pd, Pt, Fe, Ru, Os, Co, Rh, Ir, Cu,
**dadurch gekennzeichnet, dass** Schritt (a) und/oder Schritt (b) in Gegenwart einer festen Säure **S₁** durchgeführt wird.

2. Verfahren nach Anspruch 1, wobei in Schritt (a) Wasser zugefügt wird.

3. Verfahren nach Anspruch 2, wobei die Menge des in Schritt (a) zugefügten Wassers bezogen auf die Menge der Verbindung **(II)** zwischen 0.1 bis 1 molaren Äquivalenten liegt.

4. Verfahren nach einem der Ansprüche 1 bis 4, wobei zwischen Schritt (a) und Schritt (b) Wasser entfernt wird.

5. Verfahren nach Anspruch 4, wobei die Entfernung des Wassers durch Destillation erfolgt.

6. Verfahren nach einem der Ansprüche 1 bis 5, wobei es sich bei der festen Säure **S₁** um Siliciumoxid oder ein Metalloxid handelt.

7. Verfahren nach Anspruch 6, wobei die feste Säure **S₁** aus der Gruppe bestehend aus Al₂O₃, ZrO₂, SiO₂, MgO₂, TiO₂ ausgewählt ist.

8. Verfahren nach einem der Ansprüche 1 bis 7, wobei im Schritt (a) 0.25 bis 1.25 Mol an Verbindung der Formel **(II)** für jedes Mol Amin der Formel **(III-A)** eingesetzt wird, und 0.45 bis 2.5 Mol der Verbindung der Formel **(II)** für jedes Mol Amin der Formel **(III-B)** eingesetzt wird.

9. Verfahren nach einem der Ansprüche 1 bis 8, wobei der Druck in Schritt (b) im Bereich von 2 bar bis 500 bar liegt.

10. Verfahren nach einem der Ansprüche 1 bis 9, wobei die Temperatur in Schritt (b) im Bereich 20 °C bis 350 °C liegt.

11. Verfahren nach einem der Ansprüche 1 bis 10, wobei der Voll- oder Trägerkatalysator **K₁** mindestens ein elementares Metall, welches aus der Gruppe bestehend aus Pd, Pt, Ni, Co ausgewählt ist, umfasst.

12. Verfahren nach einem der Ansprüche 1 bis 11, wobei Schritt (a) in Gegenwart einer festen Säure **S₁** durchgeführt wird.

13. Verfahren nach Anspruch 12, wobei die feste Säure **S₁** und der Voll- oder Trägerkatalysator **K₁** räumlich getrennt vorliegen.

14. Verfahren nach einem der Ansprüche 1 bis 13, wobei das Amin in Schritt (a) aus der Gruppe bestehend aus Ammoniak, Dimethylamin, *n*-Butylamin, Hexamethylendiamin, 3-Dimethylaminopropylamin ausgewählt ist.

15. Verfahren nach einem der Ansprüche 1 bis 14, wobei das Verhältnis aus dem Gesamtgewicht der eingesetzten festen Säure **S₁** zum Gesamtgewicht aller eingesetzten Verbindungen der Formel **(II)** im Bereich 0.1 : 1 bis 50 : 1 liegt.
